(19) Europäisches Patentamt
European Patent Office
Office européen des brevets

(11) EP 0 501 912 B1

(12) EUROPÄISCHE PATENTSCHRIFT

(45) Veröffentlichungstag und Bekanntmachung des Hinweises auf die Patenterteilung:
**14.08.1996 Patentblatt 1996/33**

(51) Int. Cl.$^6$: **C07D 317/22**, C07D 317/72, C07F 15/00, A01N 43/28, C07F 9/50

(21) Anmeldenummer: **92810112.0**

(22) Anmeldetag: **18.02.1992**

# (54) Methyldioxolan

Methyldioxolane

Méthyldioxolane

(84) Benannte Vertragsstaaten:
**AT BE CH DE ES FR GB GR IT LI LU NL PT**

(30) Priorität: **25.02.1991 CH 572/91**

(43) Veröffentlichungstag der Anmeldung:
**02.09.1992 Patentblatt 1992/36**

(73) Patentinhaber: **CIBA-GEIGY AG**
**4002 Basel (CH)**

(72) Erfinder:
- **Karrer, Friedrich, Dr.**
  **CH-4800 Zofingen (CH)**
- **Buser, Hans-Peter, Dr.**
  **CH-4144 Arlesheim (CH)**
- **Ramos, Gerardo, Dr.**
  **CH-4144 Arlesheim (CH)**
- **Rindlisbacher, Alfred**
  **CH-4132 Muttenz (CH)**
- **Venanzi, Luigi Mario**
  **CH-8032 Zürich (CH)**
- **Ward, Thomas Raoul**
  **Ithaca, N.Y. 14850 (US)**

(56) Entgegenhaltungen:
**DE-A- 2 655 910**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 501 912 B1

## Beschreibung

Die vorliegende Erfindung betrifft das neue 2R,4S-2-Aethyl-4-[(4-phenoxyphenoxy)-methyl]-dioxolan, Verfahren zu seiner Herstellung, Schädlingsbekämpfungsmittel, welche diese Verbindung enthalten, sowie die Verwendung bei der Kontrolle von Schädlingen aus dem Kreis der Arthropoda, vorzugsweise von Insekten und Vertretern der Ordnung Akarina. Ferner betrifft die Erfindung neue Zwischenprodukte, welche für die Herstellung des neuen Wirkstoffs entwickelt wurden.

Das erfindungsgemässe 2R,4S-2-Aethyl-4-[(4-phenoxyphenoxy)-methyl]-dioxolan entspricht der Formel I

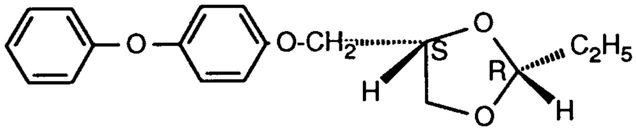

(I)

Aus der Literatur ist das Enantiomerengemisch von 2-Aethyl-4-[(4-phenoxyphenoxy)-methyl]-dioxolan beispielsweise aus der DE-OS 2 655 910 bekannt. Das Enantiomerengemisch zeichnet sich durch gute Brauchbarkeit bei der Bekämpfung von Schädlingen aus dem Kreis der Arthopoda, insbesondere der Klasse der Insekta und der Ordnung der Akarina aus. Trotz der guten Wirksamkeit befriedigten die Eigenschaften des bekannten Isomerengemisches bei der Anwendung als Schädlingsbekämpfungsmittel gegen sämtliche unerwünschte Schädlinge nicht immer vollständig. Insbesondere werden gelegentlich bei ungünstigen Klimabedingungen und bei ungewollter Überdosierung wie zum Beispiel bei Sprühstreifenüberlagerung durch Winddrift oder ungenaues Streifensprühen, phytotoxische Effekte an den behandelten Nutzpflanzenkulturen beobachtet. Es besteht deshalb weiterhin ein Bedürfnis nach einem Mittel zur Bekämpfung von Schädlingen mit verbesserten Eigenschaften.

Überraschenderweise kann dieses Bedürfnis durch den Einsatz des erfindungsgemäss vorgeschlagenen Einzelisomeren der Formel I weitgehend befriedigt werden. Es wurde nämlich festgestellt, dass das erfindungsgemässe 2R,4S-Isomere nicht nur eine gegenüber dem Enantiomerengemisch verbesserte, höhere Wirksamkeit gegenüber den unerwünschten Schädlingen aufweist, sondern ausserdem gleichzeitig überraschenderweise auch von den behandelten Pflanzen besser toleriert wird, als das Enantiomerengemisch. Bei erhöhter Wirksamkeit und verminderter Phytotoxizität ergibt sich für den Anwender eine verbreiterte Sicherheitsmarge, in der bei Bedarf die Menge an ausgebrachtem Wirkstoff erhöht werden kann, um beispielsweise auch schwer zu bekämpfende Schädlinge noch wirksam zu kontrollieren, ohne dass dabei gleichzeitig eine Schädigung der behandelten Nutzpflanzen befürchtet werden muss.

Erfindungsgemäss wird daher das 2R,4S-2-Aethyl-4-[(4-phenoxyphenoxy)-methyl]-dioxolan als Mittel zur Schädlingsbekämpfung, insbesondere zur Bekämpfung von Insekten und Vertretern der Ordnung Akarina vorgeschlagen.

Die erfindungsgemässe Verbindung der Formel I kann grundsätzlich aus dem vorbekannten Enantiomerengemisch durch geeignete Trennmethoden für Enantiomeren erhalten werden. Solche Methoden sind beispielsweise physikalische Methoden wie fraktionierte Kristallisation oder Chromatographie, gewünschtenfalls auch an chiralen stationären Phasen, sowie Derivatisierung mit definiert optisch aktiven Hilfssubstanzen und Trennung der so erhaltenen Enantiomerenpaare durch die genannten Trennverfahren. Anschliessend werden aus solchen isolierten Enantiomer-Derivaten durch Abspaltung der Hilfssubstanz die reinen optischen Antipoden erhalten. In der Praxis ist es jedoch meist vorteilhaft, das gewünschte Einzelisomer durch gezielte stereoselektive Synthese herzustellen.

Durch derartige gezielte Synthesewege erhält man die Verbindung der Formel I beispielsweise, indem man entweder

a) das Bromäthyldioxolan der Formel II

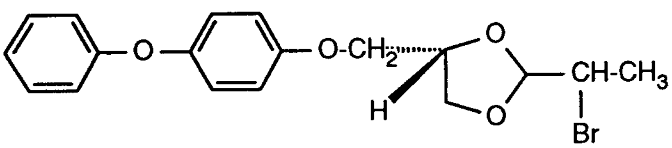

(II)

in Gegenwart von tert.Butanol und Kalium-tert.butylat dehydrobromiert und das erhaltene Ketenacetal der Formel III

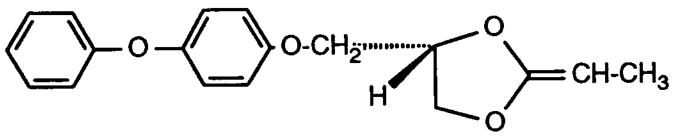

(III)

mit Wasserstoff in Gegenwart eines Palladium/Calciumcarbonat-Katalysators hydriert, oder
b) das Diol der Formel IV

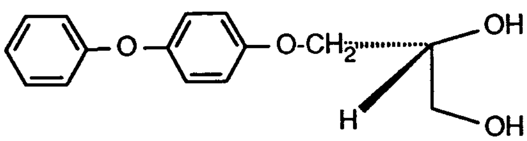

(IV)

in Gegenwart des stereoselektiven Katalysators der Formel V

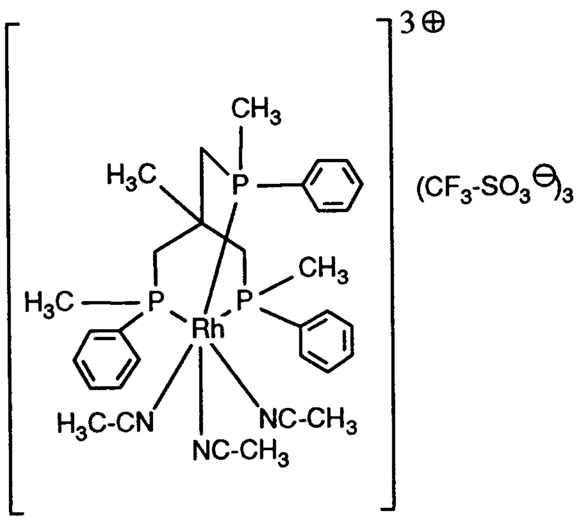

(V)

mit einem Propionaldehydacetal der Formel VI

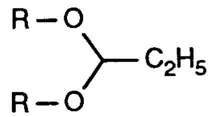

(VI)

worin R für $C_1$-$C_4$-Alkyl, vorzugsweise Methyl oder Aethyl steht, umsetzt.

Vorzugsweise werden die beiden obigen Verfahren in geeigneten inerten organischen Lösungsmitteln durchgeführt. Für das Verfahren a) eignen sich besonders Alkohole wie Methanol, Aethanol, Isopropanol oder insbesondere tert.Butanol. Für das Verfahren b) kommen aromatische Kohlenwasserstoffe wie Toluol, Xylol, Mesitylen, Benzol oder Tetralin, oder halogenierte Kohlenwasserstoffe wie Methylenchlorid, Chloroform, Trichloräthan oder Tetrachloräthan in Frage.

Die Verfahrensvarianten a) und b) liefern das Produkt der Formel I in einer sehr hohen optischen Reinheit. Enantiomere mit unerwünschter Orientierung der Substituenten an den beiden asymmetrischen Kohlenstoffatomen des Dioxolanringes entstehen nur in sehr geringen Anteilen. Typische Isomerenreinheitsgrade betragen beim Verfahren a) 94 % und beim Verfahren b) mindestens 90 %.

Das Verfahren a) wird in einer typischen Reaktionsführung so ausgeführt, dass man das Bromäthyldioxolan der Formel II zusammen mit zwei Äquivalenten Kalium-tert.butylat in trockenem tert.Butanol suspendiert und für ungefähr

4 Stunden auf eine Temperatur zwischen +80°C und +100°C erhitzt. Nach dem Abkühlen enthält diese Lösung das Ketenacetal der Formel III. Um Verluste dieser Verbindung durch Hydrolyse und Zersetzung bei der Isolierung zu vermeiden, setzt man das erhaltene Reaktionsprodukt ohne weiteren Reinigungsschritt in einem Autoklaven in Gegenwart eines 2 % bis 15 %, vorzugsweise 5%igen Palladium- auf Calciumcarbonat- Katalysators mit Wasserstoffgas um. Typische Reaktionsbedingungen für diesen Hydrierschritt sind 80 bar bis 150 bar bei Temperaturen zwischen +15°C und +30°C. Man erhält das Produkt der Formel I in 2R,4S-Konfiguration nach der Aufarbeitung in einem Reinheitsgrad von 94 %. 6 % des erhaltenen Reaktionsprodukts hat 2R,4R-Konfiguration.

In der typischen Ausführungsform wird das Verfahren b) so durchgeführt, dass man das Diol der Formel IV zusammen mit dem Propionaldehydacetal der Formel VI äquimolar in einem geeigneten Lösungsmittel vorlegt und den Katalysator der Formel V in einer Menge von ungefähr 0,01 bis 0,0001, vorzugsweise 0,0005 Moläquivalenten zufügt. Bei einer Reaktionstemperatur zwischen +20°C und +30°C betragen die Reaktionszeiten bis zum vollständigen Umsatz der Ausgangsmaterialien bis zu 48 Stunden. Man erhält nach der Aufarbeitung das Produkt der Formel I in einem Reinheitsgrad von gösser als 90 % des 2R,4S-Isomeren. Der Anteil des ebenfalls gebildeten 2R,4R-Isomeren liegt unter 10 %.

Die Ausgangsmaterialien und Zwischenprodukte der Formeln II, III und IV sind speziell für die Synthese des Wirkstoffs der Formel I entwickelt. Sie bilden daher einen zusätzlichen Aspekt der vorliegenden Erfindung. Die Zwischenprodukte der Formel VI sind bekannt und im Handel erhältlich. Ebenfalls neu und speziell für das Syntheseverfahren b) entwickelt worden ist der Acetalisierungskatalysator der Formel V.

Die Verbindung der Formel II erhält man in einer Acetalisierungsreaktion, indem man das Diol der Formel IV in Gegenwart eines sauren Katalysators mit einem α-Brompropionaldehydacetal der Formel VII

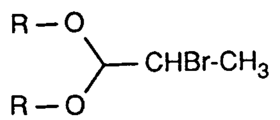

(VII)

worin R für $C_1$-$C_4$-Alkyl, vorzugsweise Methyl oder Aethyl steht, umsetzt.

Die Umsetzung (IV + VII → II) führt man unter den für eine Acetalisierung üblichen Bedingungen aus. Vorzugsweise wählt man ein inertes organisches Lösungsmittel wie einen aromatischen Kohlenwasserstoff, beispielsweise Toluol oder Xylol, und erhitzt die Reaktionspartner in äquimolaren Mengen in der Gegenwart eines sauren Katalysators bis zum vollständigen Umsatz der Ausgangsmaterialien auf eine Temperatur, welche dem Siedepunkt des Reaktionsgemisches entspricht. Als saure Katalysatoren kommen dabei sowohl starke anorganische Säuren wie HCl, $H_2SO_4$, $H_3PO_4$, als auch organische Säuren wie $F_3C$-COOH, $F_3C$-$SO_3$-H, $H_3C$-$C_6H_4$-$SO_3H$ und saure Ionenaustauscher in Frage.

Gewünschtenfalls kann das durchlaufene Zwischenprodukt der Formel III nach dem ersten Verfahrensschritt der Verfahrensvariante a) aus dem Reaktionsgemisch isoliert werden, indem man das Reaktionsgemisch nach der Eliminierung von HBr unter Ausschluss von Wasser aufarbeitet.

Das Diol der Formel IV ist auf vorteilhafte Weise nach den beiden folgenden Verfahren erhältlich.

Nach einem ersten Verfahren erhält man das Diol der Formel IV, indem man 4-Phenoxyphenol der Formel VIII

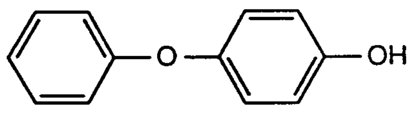

(VIII)

in Gegenwart einer Base wie Kalium-tert.butylat in einem polaren Lösungsmittel wie Dimethylsufoxid mit 4R-2,2-Dimethyl-4-tosyloxymethyl-dioxolan der Formel IX

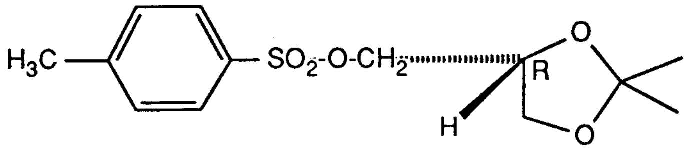

(IX)

umsetzt, und den erhaltenen 4-Phenoxyphenyl-methyläther der Formel X

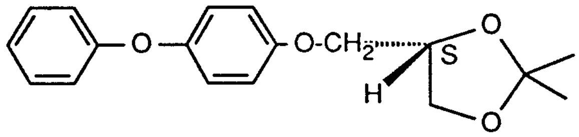

(X)

einer Hydrolyse in Gegenwart eines sauren Katalysators unterwirft.

Die Hydrolysereaktion (X → IV) führt man vorzugsweise in einem Lösungsmittel wie Methanol, Aethanol, Isopropanol oder Wasser durch. Die Reaktionstemperaturen liegen bei beiden Reaktionsschritten (VIII + IX → X und X → IV) im allgemeinen zwischen +10°C und +30°C. Als sauere Katalysatoren können anorganische oder organische Säuren eingesetzt werden. Beispiele sind HCl, $H_2SO_4$, $H_3PO_4$, $CF_3SO_3H$, $F_3C\text{-}COOH$. Ferner können saure Ionenaustauscher als saure Katalysatoren verwendet werden. Beide Reaktionsschritte verlaufen in der Regel mit Ausbeuten zwischen 90-95 % der Theorie. Die optische Reinheit des Produkts der Formel IV ist besser als 95 %.

In einem zweiten Verfahren erhält man das Diol der Formel IV, indem man racemisches Glycerin mit einem Camphersulfonamid der Formel XI

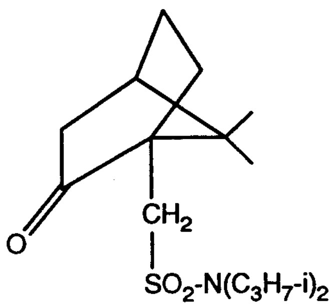

(XI)

in Gegenwart eines sauren Katalysators umsetzt, aus dem erhaltenen Reaktionsgemisch das kristallisierende Einzeldiastereomere der Formel XII

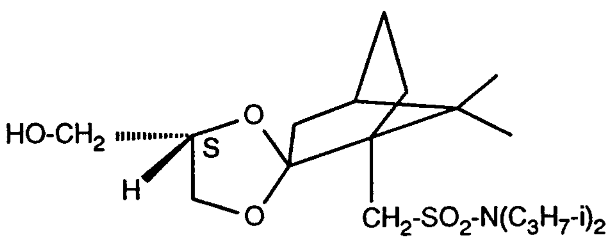

(XII)

isoliert, dieses in Gegenwart einer Base mit Methansulfonsäurechlorid in das Mesylat der Formel XIII

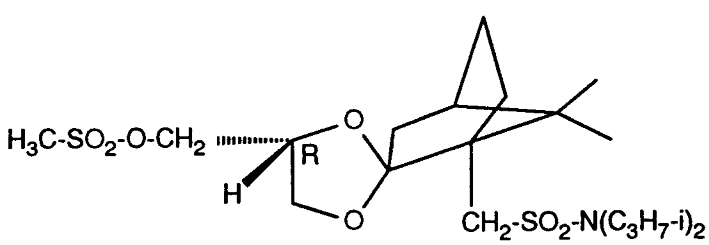

(XIII)

überführt, dieses Zwischenprodukt in Gegenwart einer Base mit 4-Phenoxyphenol der Formel VIII umäthert, und das erhaltene Addukt der Formel XIV

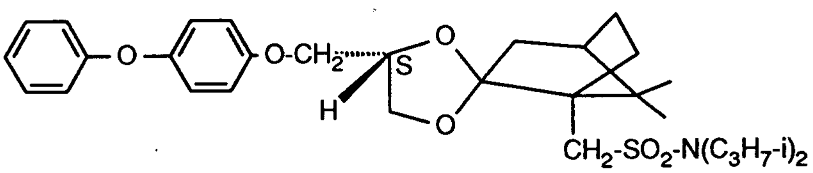

(XIV)

in Gegenwart einer Säure hydrolysiert.

Der erste Reaktionsschritt dieses Verfahrens zur Herstellung des Diols der Formel IV, entspricht in seiner Ausführung den üblichen Bedingungen für eine Acetalisierungsreaktion (XI → XII). So lässt sich dieser Kondensationsschritt in Gegenwart einer Säure wie p-Toluolsulfonsäure, Campher-10-sulfonsäure oder einem sauren Ionenaustauscherharz, in einem wasserfreien Lösungsmittel wie beispielsweise Toluol oder Benzol bei azeotroper Destillation, durchführen. Aus dem Gemisch der erhaltenen Acetale lässt sich das 4S-Einzeldiastereomere der Formel XII leicht dadurch abtrennen und identifizieren, dass es aus dem Diastereomerengemisch auskristallisiert. Es weist einen Schmelzpunkt von 98-100°C auf. Der Dritte Reaktionsschritt (XIII → XIV) erfolgt unter üblichen Bedingungen einer $S_N$-Reaktion unter Anwesenheit einer Base. Als besonders geeignet hat sich im vorliegenden Verfahrensschritt die Verwendung von Kaliumcarbonat als Base erwiesen. Im vierten Reaktionsschritt (XIV → IV) erfolgt die Hydrolyse des Acetalskörper unter üblichen Bedingungen in Gegenwart einer Säure wie HCl, $H_2SO_4$, $H_3PO_4$ in Wasser oder einem wässrigen Reaktionsmedium von Methanol, Aethanol oder Isopropanol. Man erhält das Diol der Formel IV in dieser Verfahrensvariante mit einem optischen Reinheitsgrad von 98 % und einem Schmelzpunkt von 88,4°C-89,5°C. Die optische Drehung beträgt $[\alpha]_D^{RT}$ = -6,34°. Neben dem Diol der Formel IV wird im letzten Schritt der Reaktionsfolge das Camphersulfonamid der Formel XI zurückerhalten, sodass es bei einer Wiederholung der Reaktion zur Herstellung von Verbindung IV wiederverwendet werden kann.

Die Ausgangsmaterialien der Formeln VII, VIII, IX und XI sind bekannt und im Handel erhältlich. Die durchlaufenen Zwischenprodukte der Formeln X und XIV sind neu. Sie wurden speziell für die Synthese des Wirkstoffs der Formel I entwickelt und bildden daher einen weiteren Aspekt der vorliegenden Erfindung.

Der neue stereoselektive Katalysator der Formel V wurde ebenfalls für die Synthese des Wirkstoffs der Formel I speziell entwickelt. Man erhält diesen stereoselektiven Acetalisierungskatalysator, indem man 3 Äquivalente Methylphenylphosphin-Lithium der Formel XV

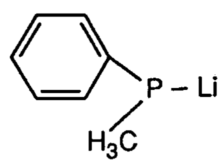

(XV)

mit Trichlorneopentan der Formel XVI

$H_3C\text{-}C(CH_2Cl)_3$ (XVI)

umsetzt, das erhaltene Triphosphin der Formel XVII

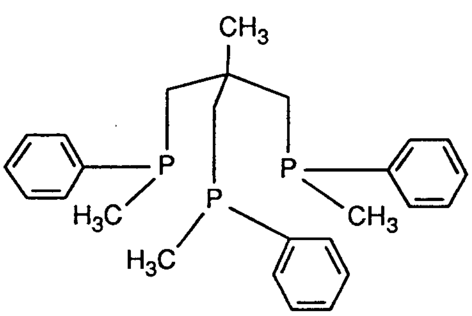

(XVII)

mit Bicyclo[2.2.1]hepta-2,5-dien-rhodium(1)-chlorid-Dimer der Formel XVIII

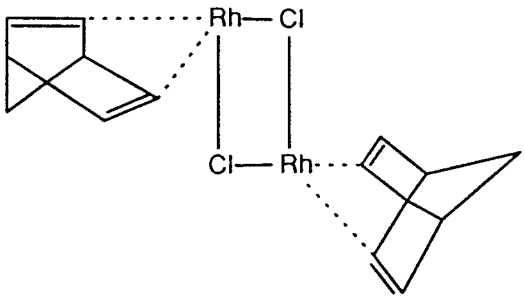

(XVIII)

und Silbertrifluormethansulfonat ($F_3C$-$SO_2$-O-Ag) in einen Komplex der Formel XIX

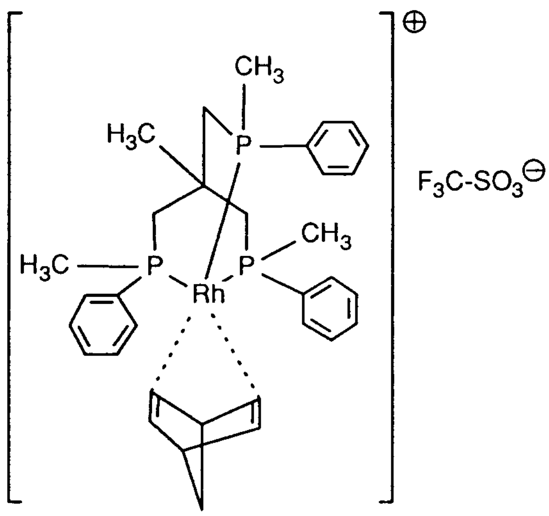

(XIX)

überführt, und dieses Vorprodukt in Acetonitril mit Trifluormethansulfonsäure behandelt.

Die Ausgangsmaterialien der Formeln XV, XVI und XVIII zur Herstellung des Katalysators V sind aus der Literatur bekannt. Die Zwischenprodukte der Formeln XVII und XIX sind neu und wurden speziell für die Synthese des neuen Katalysators der Formel V entwickelt.

Es wurde nun gefunden, dass die erfindungsgemässe Verbindung der Formel I bei günstiger Warmblüter-, Fisch- und Pflanzenverträglichkeit ein wertvoller Wirkstoff in der Schädlingsbekämpfung ist. Insbesondere betrifft die Anwendung des erfindungsgemässen Wirkstoffs gegen Insekten und Spinnentiere, die in Nutz- und Zierpflanzen in der Landwirtschaft, insbesondere in Baumwoll-, Gemüse- und Obstpflanzungen, im Forst, im Vorrats- und Materialschutz sowie im Hygienesektor insbesondere an Haus-und Nutztieren vorkommen. Sie ist gegen alle oder einzelne Entwicklungsstadien von normal sensiblen aber auch resistenten Arten wirksam. Dabei kann sich ihre Wirkung in unmittelbarer Abtötung der Schädlinge oder erst nach einiger Zeit, beispielsweise bei einer Häutung, oder in einer verminderten Eiablage und/oder Schlupfrate zeigen. Zu den oben erwähnten Schädlingen gehören:
aus der Ordnung Lepidoptera zum Beispiel
Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp., Dermestes

spp., Diabrotica spp., Epilachna spp., Eremnus spp., Leptinotarsa decemlineata, Lissorhoptrus spp. Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;
aus der Ordnung der Orthoptera zum Beispiel
Blatta spp., Blattella spp., Gryllotalpa spp., Leucophaea maderae, Locusta spp., Periplaneta spp. und Schistocerca spp.;
aus der Ordnung der Isoptera zum Beispiel
Reticulitermes spp.;
aus der Ordnung der Psocoptera zum Beispiel
Liposcelis spp.;
aus der Ordnung der Anoplura zum Beispiel
Haematopinus spp., Linognathus spp. Pediculus spp., Pemphigus spp. und Phylloxera spp.;
aus der Ordnung der Mallophaga zum Beispiel
Damalinea spp. und Trichodectes spp.;
aus der Ordnung der Thysanoptera zum Beispiel
Frankliniella spp., Hercinothrips spp., Taeniothrips spp., Thrips palmi, Thrips tabaci und Scirtothrips aurantii;
aus der Ordnung der Heteroptera zum Beispiel
Cimex spp., Distantiella theobroma, Dysdercus spp., Euchistus spp. Eurygaster spp. Leptocorisa spp., Nezara spp., Piesma spp., Rhodnius spp., Sahlbergella singularis, Scotinophara spp. und Triatoma spp.;
aus der Ordnung der Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporiorum, Trioza erytreae und Unaspis citri;
aus der Ordnung der Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung der Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp. Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung der Siphonaptera zum Beispiel
Ceratophyllus spp., Xenopsylla cheopis,
aus der Ordnung der Acarina zum Beispiel
Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp.; und
aus der Ordnung der Thysanura zum Beispiel
Lepisma saccharina.

Die Verbindung der Formel I eignet sich besonders zur Bekämpfung von Schädlingen in Obst- und Zitruskulturen. Insbesondere werden Schildläuse wie Aonidiella aurantii, Saissetia olea, Lepidosaphes beckii, Quadraspidiotus perniciousus, Planococcus citri, Unaspis citri, Ceroplastes floridensis, Ceroplastes sinensis, Parlatoria pergandei und Lepidosaphes ulmi und Obstschädlinge wie Adoxophyes orana, Cydia pomonella, Psylla piricola, Leucoptera scitella und Lobesia botrana wirkungsvoll bekämpft. Ebenfalls gute Wirkungen werden gegen den Reisschädling Nilaparvata lugens und gegen Zecken wie Boophilus microplus beobachtet.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindung und der sie enthaltenden Mittel lässt sich durch Zusatz von anderen Insektiziden und/oder Akariziden wesentlich verbreitern und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindung der Formel I wird in unveränderter Form oder vorzugsweise zusammen mit den in der Formulierungstechnik üblichen Hilfsmitteln eingesetzt und können daher beispielsweise zu emulgierbaren Konzentraten, direkt versprüh- oder verdünnbaren Lösungen, verdünnten Emulsionen, Spritzpulvern, löslichen Pulvern, Stäubemitteln, Granulaten, auch Verkapselungen in polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt. Ferner eignen sich die Verbindungen der Formel I auch für den Einsatz bei der Behandlung von Saatgut. Dabei kann sowohl das Saatgut vor dem Säen mit dem Wirkstoff oder einer den Wirkstoff enthaltenden Formulierung behandelt oder gebeizt werden, als auch der Wirkstoff beim Säen in die Saatfurche appliziert werden.

Die Formulierung, das heisst die den Wirkstoff der Formel I, beziehungsweise Kombinationen dieses Wirkstoffs mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, zum Beispiel durch inniges Vermischen und/oder Vermahlen der Wirkstoffe mit Streckmitteln, wie beispielsweise mit Lösungsmitteln, festen Trägerstoffen, und gegebenenfalls obeflächenaktiven Verbindungen (Tensiden).

Als Lösungsmittel können in Frage kommen: Aromatische Kohlenwasserstoffe, bevorzugt die Fraktionen $C_8$ bis $C_{12}$ von Akylbenzolen wie Xylolgemische oder älkylierte Naphthaline, aliphatische oder cycloaliphatische Kohlenwasserstoffe, wie Cyclohexan, Paraffine oder Tetrahydronaphthalin, Alkohole wie Aethanol, Propanol oder Butanol, und Glykole sowie deren Äther und Ester, wie Propylenglykol, Dipropylenglykoläther, Äthylenglykol, Äthylenglykolmonomethyl- oder -äthyläther, Ketone, wie Cyclohexanon, Isophoron oder Diacetanolakohol, starke polare Lösungsmittel, wie N-Methyl-2-pyrrolidon, Dimethylsulfoxid oder Dimethylformamid oder Wasser, Pflanzenöle, wie Raps-, Rizinus-, Kokosnuss- oder Sojaöl; gegebenenfalls auch Silikonöle.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als obeflächenaktive Verbindungen kommen je nach Art des zu formulierenden Wirkstoffs der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische obeflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie die Natrium- oder Kalium-Salze der Öl- oder Stearinsäure, oder von natürlichen Fettsäuregemischen, die beispielsweise aus Kokosnuss- oder Tallöl gewonnen werden können. Ferner sind als Tenside auch die Fettsäuremethyl-taurin-salze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, beispielsweise das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie zum Beispiel Salze des Phosphorsäureesters eines p-Nonylphenol-(4-14)-Äthylenoxid-Adduktes oder Phospholipide in Frage.

Als nichtionische Tenside kommen in erster Linie Polyglykolätherderivate von aliphatischen oder cycloaliphatischen Akoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Äthylenglykoläthergruppen und 10 bis 100 Propylenglykoläthergruppen enthaltenden Polyäthylenoxid-Addukte an Polypropylenglykol, Äthylendiaminopolypropylenglykol und Akylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykol-Einheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Oktylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Akyl-, Benzyl- oder niedrige Hydroxyalkylreste aufweisen. Die Salze liegen vorzugsweise als Halogenide, Methylsulfate oder Äthylsulfate vor, beispielsweise das Stearyltrimethylammoniumchlorid oder das Benzyl-di-(2-chloräthyl)-äthylammoniumbromid.

Die in der Formulierungstechnik gebräuchlichen Tenside sind beispielsweise in folgenden Publikationen beschrieben:
"Mc Cutcheon's Detergents and Emulsifiers Annual", Mc Publishing Corp., Glen Rock, NJ, USA, 1988",
H. Stache, "Tensid-Taschenbuch", 2. Aufl., C. Hanser Verlag München, Wien 1981,
M. and J. Ash. "Encyclopedia of Surfactants", Vol. I-III, Chemical Publishing Co., New York, 1980-1981.

Die pestiziden Zubereitungen enthalten in der Regel 0,1 bis 99 %, insbesondere 0,1 bis 95 % Wirkstoff der Formel I oder Kombinationen dieses Wirkstoffs mit andern Insektiziden oder Akariziden, 1 bis 99,9 % eines festen oder flüssigen Zusatzstoffs und 0 bis 25 %, insbesondere 0,1 bis 25 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000 g Wirkstoff pro Hektar, vorzugsweise 25 bis 500 g/ha.

Insbesondere setzen sich bevorzugte Formulierungen folgendermassen zusammen: (% = Gewichtsprozent)

| Emulgierbare Konzentrate: | |
|---|---|
| Aktiver Wirkstoff: | 1 bis 90 %, bevorzugt 5 bis 20 % |
| oberflächenaktives Mittel: | 1 bis 30 %, vorzugsweise 10 bis 20 % |
| flüssiges Trägermittel: | 5 bis 94 %, vorzugsweise 70 bis 85 % |

| Stäube: | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

| Suspension-Konzentrate: | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

| Benetzbare Pulver: | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

| Granulate: | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

Die Mittel können auch weitere Zusätze wie Stabilisatoren, z.B. gegebenenfalls epoxidierte Pflanzenöle (epoxidiertes Kokosnussöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Sie schränken die Erfindung nicht ein.

Beispiel H1: 2R,4S-2-Aethyl-4-[(4-phenoxyphenoxy)-methyl]-dioxolan

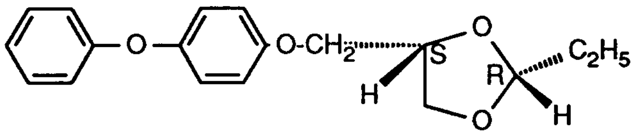

a) 2RS,4S-2-(1-Bromäthyl)-4-[(4-phenoxyphenoxy)-methyl]-dioxolan

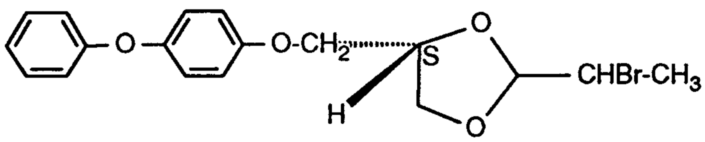

167 g 2R-2,3-Dihydroxy-1-(4-phenoxyphenoxy)-propan, 162,5 g 2-Brompropionaldehyddiäthylacetal und 167 g saures Ionenaustauscherharz (DOWEX 50W.8, 16-40 mesh, $H^{\oplus}$-Form) werden mit 1,3 l Toluol gemischt und für 5,5 Stunden zum Rückfluss erhitzt. In diesem Zeitraum destilliert man 700 ml eines Azeotrops aus Aethanol und Toluol aus dem Gemisch ab. Nach Abkühlen auf Raumtemperatur wird das Ionentauscherharz abfiltriert und das Filtrat eingedampft. Der Rückstand wird über 1,2 kg Kieselgel mit einem Laufmittelgemisch aus Hexan/Aethylacetat (5:1) chromatographiert. Man erhält 223,3 g 2RS,4S-2-(1-Bromäthyl)-4-[(4-phenoxyphenoxy)-methyl]-dioxolan als leicht gelbliches Oel, $n_D^{20}$: 1,5691.

b) 4S-2-Aethyliden-4-[(4-phenoxyphenoxy)-methyl]-dioxolan

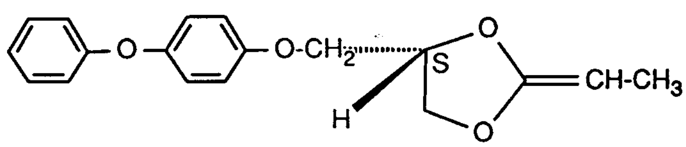

Eine Suspension aus 50 g 2RS,4S-2-(1-Bromäthyl)-4-[(4-phenoxyphenoxy)-methyl]-dioxolan, 29,65 g frisch sublimiertem Kalium-tert.butylat und 500 ml frisch über CaO destilliertem tert.Butanol wird für 3,5 Stunden bei einer Temperatur von +95°C unter einer Argonatmosphäre gerührt. Nach dem Abkühlen auf +25°C, setzt man dieses Gemisch direkt in den folgenden Reaktionsschritt ein.

c) Das unter Beispiel H1 b) erhaltene Reaktionsgemisch wird mit weiteren 300 ml tert.Butanol unter Gasabschluss in einen Autoklaven gespült. Nach Zusatz von 31,4 g 5%igem getrockneten Palladium-auf-Calciumcarbonat -Katalysator wird Wasserstoffgas bis zu einem Druck von 120 bar aufgedrückt. Der Reaktor wird für 38 Stunden bei +30°C gehalten. Anschliessend wird das Reaktionsgemisch mit 150 ml Aethanol verdünnt und über Diatomerenerde filtriert. Das Filtrat wird eingedampft, in Aethylacetat aufgenommen, mit Wasser gewaschen, über Natriumsulfat getrocknet und eingedampft. Die erhaltenen 38,3 g Rohprodukt werden an 500 g Kieselgel mit einem Hexan/Diäthyläthergemisch (9:1) als Laufmittel chromatographisch gereinigt. Man erhält 22,8 g des Produkts mit einem optischen Reinheitsgrad von 94 % des 2R,4S-Enantiomeren, sowie eine weitere Fraktion von 8,55 g des Produkts mit einem Reinheitsgrad von weniger als 90 %.

<u>Beispiel H2:</u> <u>2R-2,3-Dihydroxy-1-(4-phenoxyphenoxy)-propan</u>

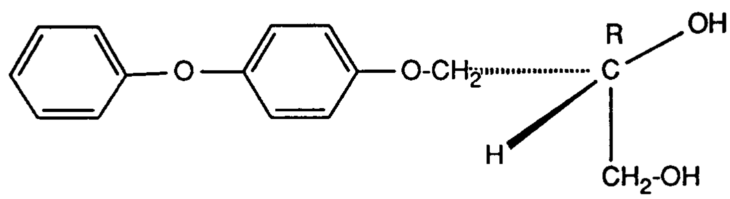

a) 4S-2-Spiro[(10-Diisopropylaminosulfonyl)-2-bornan]-4-hydroxymethyl-dioxolan

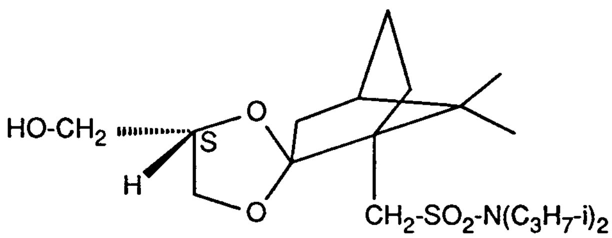

4,8 g 10-Diisopropylaminosulfonylcampher, 1,6 ml Glycerin und 230 mg p-Toluolsulfonsäure-Monohydrat werden in 90 ml Benzol aufgenommen. Die Suspension wird für 37 Stunden am Wasserabscheider gekocht. Wenn mittels Dünnschichtchromatogramm eine unvollständige Reaktion festgestellt wird, setzt man nochmals 1,6 ml Glycerin und 234 mg p-Toluolsulfonsäure zu und kocht es weitere 23 Stunden am Wasserabscheider.

Durch Eindampfen des Reaktionsgemisches und Chromatographie des Rückstandes an Kieselgel mit einem Hexan/Aethylacetat-Gemisch (4:1) erhält man neben 1,63 g eines Diastereomerengemisches 1,45 g des diastereomerenreinen 4S-2-Spiro[(10-Diisopropylaminosulfonyl)-2-bornan]-4-hydroxymethyl-dioxolans in kristalliner Form mit einem Schmelzpunkt von 98-100°C. Die absolute Konfiguration des Spiro-Kohlenstoffatoms wurde nicht determiniert.

b) 4R-2-Spiro[(10-Diisopropylaminosulfonyl)-2-bornan]-4-methylsulfonyloxymethyl-dioxolan

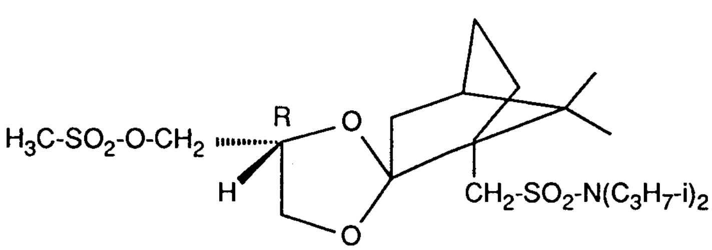

Eine Lösung von 482,5 mg 4S-2-Spiro[(10-Diisopropylaminosulfonyl)-2-bornan]-4-hydroxymethyl-dioxolan in 10 ml Methylenchlorid wird bei 0°C zuerst mit 270 µl Triäthylamin und dann mit 120 µl (1,54 mmol) Methansulfonylchlorid versetzt. Nach 5-stündigem Rühren bei Raumtemperatur wird die leicht gelbliche Reaktions-Lösung auf 20 ml 1 N wässrige Salzsäure gegossen und mit 40 ml Methylenchlorid verdünnt. Die organische Phase wird zweimal

mit je 20 ml 1 N Salzsäure extrahiert. Alle wässrigen Phasen werden mit 2 x 50 ml Methylenchlorid gewaschen. Die vereinigten organischen Phasen werden über Natriumsulfat getrocknet und eingeengt. Nach dem Trocknen in Vakuum erhält man 580 mg diastereomerenreines 4R-2-Spiro[(10-Diisopropylaminosulfonyl)-2-bornan]-4-methylsulfonyloxymethyl-dioxolan in Form eines farblosen Öls, welches beim Stehenlassen kristallisiert, Smp. 60,5-63,5°C.

c) 4S-2-Spiro[(10-Diisopropylaminosulfonyl)-2-bornan]-4(4-phenoxyphenoxymethyl)-dioxolan

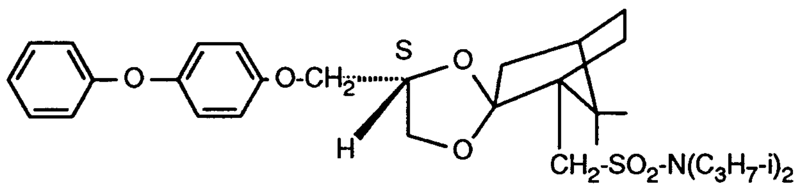

1,5 g 4R-2-Spiro[(10-Diisopropylaminosulfonyl)-2-bornan]-methylsulfonyloxymethyl-dioxolan, 720 mg 4-Phenoxyphenol und 970 mg wasserfreies Kaliumcarbonat werden in 10 ml Dimethylsulfoxid aufgenommen, worauf für 20 Stunden bei 100°C gerührt wird. Anschliessend wird das Reaktionsgemisch auf Wasser gegossen und dreimal mit Aether extrahiert. Die Aetherphasen werden mit Wasser und Sole gewaschen, vereinigt über Magnesiumsulfat getrocknet und eingeengt. Durch Chromatographie des öligen Rückstands an Kieselgel mit Hexan/Aethylacetat - Gemisch (8:1) erhält man 1,44 g 4S-2-Spiro[(10-Diisopropylaminosulfonyl)-2-bornan]-4-(4-phenoxyphenoxymethyl)-dioxolan in Form eines fablosen Öls.

d) 2,04 g 4S-2-Spiro[(10-Diisopropylaminosulfonyl)-2-bornan]-4-(4-phenoxyphenoxymethyl)-dioxolan werden in 25 ml Methanol gelöst und bei Raumtemperatur mit 10 ml 4 N wässriger Salzsäure versetzt. Die sofort milchig-trübe Suspension wird für 16 Stunden bei +50°C gerührt. Anschliessend wird das Reaktionsgemisch mit einer 9:1-Mischung aus Methanol und Wasser verdünnt und 3x mit Cyclohexan extrahiert. Die Cyclohexan-Phasen werden zweimal mit 75 ml einer 9:1-Mischung aus Methanol und Wasser gewaschen, vereinigt, über Magnesiumsulfat getrocknet und eingeengt. Als Rückstand erhält man 920 mg farbloses Öl. Die Methanol-Wasser-Phasen werden vereinigt und vollständig eingedampft. Der Rückstand wird in Wasser aufgenommen, und dreimal mit Aether extrahiert. Die Aetherphasen werden mit Sole gewaschen vereinigt, über Magnesiumsulfat getrocknet und eingeengt. So werden als Rückstand weitere 2,1 g farbloses Öl erhalten.

Die beiden öligen Rückstände werden vereinigt (insgesamt 3,02 g) und in siedendem Hexan verrührt. Unter starkem Rühren wird anschliessend langsam auf 0°C abgekühlt und das gebildete Kristallisat abfiltriert. Nach dem Trocknen im Vakuum erhält man 797 mg optisch reines (mehr als 98 % ee) des (-)-drehenden 2R-2,3-Dihydroxy-1-(4-phenoxyphenoxy)-propans, Smp. 88,4-89,5°C, $[\alpha]^{N}_{589}$ = - 6,34°.

Durch vollständiges Eindampfen der Mutterlauge erhält man 1,08 g des eingesetzen 10-Diisopropylaminosulfonylcamphers in Form eines Öls zurück.

Beispiel H3: 2R,4S-2-Aethyl-4-[(4-phenoxyphenoxy)-methyl-dioxolan

a) $\alpha$, $\alpha'$, $\alpha''$-Tris(methylphenylphoshino)-neopentan

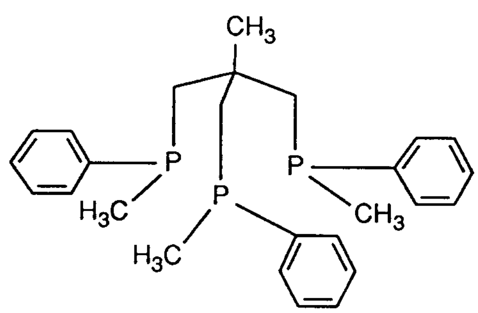

In einem 1 Liter-Dreihalskolben werden 400 ml einer Lösung von 12 g Methylphenylphosphin in entgastem Tetrahydrofuran auf -78°C gekühlt. Innerhalb von 20 Minuten lässt man 66,2 ml Butyllithium dazutropfen und rührt für 1 Stunde bei -78°C. Nach Aufwärmen auf Raumtemperatur lässt man innerhalb von 35 Minuten 5,62 g (0,032

mol) α,α',α''-Trichlorneopentan dazutropfen. Anschliessend wird die Mischung für 2 Stunden zum Rückfluss erhitzt. Es werden 250 ml entgaster Aether und 50 ml entgastes Wasser zur Lösung gegeben und die organische Phase abgetrennt und mit Magnesiumsulfat getrocknet. Nach Abdampfen des Lösungsmittels am Wasserstrahlvakuum erhält man 12,14 g α,α',α''-Tris(methylphenylphosphino)-neopentan als Rohprodukt in Form eines hellgelben Öls.

Die Reinigung erfogte indem man das Rohprodukt mit 250-300 ml entgastem Methanol unter Rückfluss rührt, bis die Lösung klar hellgelb wird. Es wird auf -78°C abgekühlt und die Methanollösung vom gefrorenen Öl abgezogen. Dabei resultiert das analysenreine Produkt als klares Öl, Ausbeute 8,77 g.

| Elementaranalyse berechnet für $C_{26}H_{33}P_3$ (438,47): | | | |
|---|---|---|---|
| | C: 71,22; | H:7,59; | P:21,19 |
| gefunden | C: 71,28; | H:7,58; | P:21,45 |

1H-NMR (200 MHz, $CDCl_3$): 7,59-7,26 (m, Ph),
2,30-1,78 (m, 2J (P,H) = 3,7Hz, $PCH_2$)
1,34-1,23 (3xd,2 (P,H) = 3,7Hz, $PCH_3$, RRS/SSR, RSR/SRS, SRR/RSS)
1,16 (d, 2J(P,H) = 3,7Hz, $PCH_3$, RRR/SSS)
1,03 (s, $CH_3$, RRR/SSS)
1,01 (s, $CH_3$, RRS/SSR, RSR/SRS, RSS/SRR)

31P-NMR (200 MHz, $CDCl_3$): -44.89 (S, RRR/SSS)
-45,08 (s, RRS/SSR, RSR/SRS, RSS/SRR).

13C-NMR (200 MHz, $CDCl_3$): 141.6 (d, 1J(P,C) = 11,9Hz, PC1) 132,3 (d, 2J (P,C) = 19,9Hz, PC2), 128,5 (d, 3J (P,C) = 6Hz, PC3), 128,3 (s, PC4), 46,0 (d.t, 1J (P,C) = 14Hz, 4J (P,C) = 8Hz, $PCH_2$), 38,4 (q, 3J (P,C) = 12,6Hz, $CH_3$), 29,1 (d, 2J P,C) = 9Hz, C), 14,6 d, 1J (P,C) = 12Hz, $PCH_3$).

b) α,α',α''-Tris(methylphenylphoshino)-neopentan-norbonandien-rhodium -(I)-trifluormethyansulfonat-Komplex

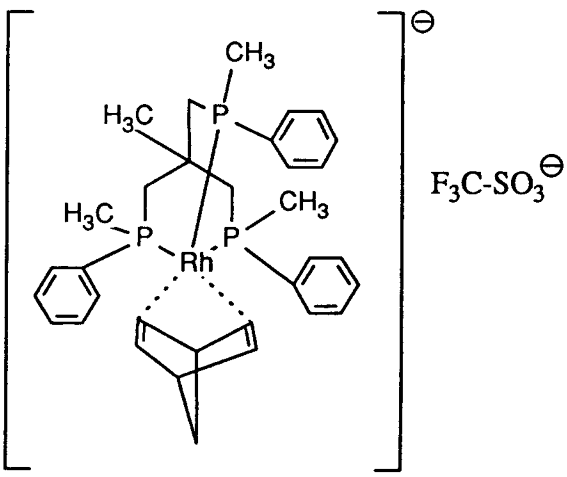

In einen 250 mi Zweihalskolben werden 990 mg α,α',α''-Tris(methylphenylphosphino)-neopentan in 20 ml entgastes Aceton gegeben und auf -78°C abgekühlt. In 100 ml entgastem Aceton werden 521 mg dimerer Rhodium(I)-norbonadien-chloro-Komplex und 581 mg Silber-trifluormethansulfonat (Ag-O-$SO_2$-$CF_3$) gelöst und für 20 Minuten gerührt. Bei -78°C wird diese Lösung über ein Papierfilter zur Phosphinlösung gegeben. Es wird auf Raumtemperatur aufgäwarmt und das Lösungsmittel abgedampft. Die Rohausbeute des resultierenden α,α',α''-Tris(methylphenylphosphino)-neopentan-norbonadien-rhodium-(I)-trifluormethansulfonat-Komplexes in Form eines tiefgelben Pulvers beträgt 1,74 g.

Die Umkristallisation erfolgt durch Lösen des Rohprodukts in 23 ml Aceton und Zugabe von 18 ml Aether, der vorsichtig über 15 Minuten zur gerührten Lösung gegeben wird. Die Lösung wird nach weiteren 15 Minuten leicht trüb und wird zur Kristallisation für 18 Stunden auf 0°C abgekühlt. Das ausgefallene orange Pulver wird abfiltriert und mit Pentan gewaschen. Ausbeute: 360 mg analysenreines Produkt.

Die Mutterlauge wird bis auf etwa 20 ml Aceton konzentriert und dann mit Pentan bis zur Fallung versetzt, abfiltriert und mit Pentan gewaschen. Man erhält so weitere 1,06 g des Produkts.

| Elementaranalyse berechnet für $C_{34}H_{41}O_3F_3P_3SRh$ (782,58); | | |
|---|---|---|
| | C: 52,18 | H: 5,28 |
| gefunden | C: 51,68 | H: 5,32 |

| | |
|---|---|
| 31P-NMR (101 MHz, $CDCl_3$): | 14,2 (d.t,1J(Rh,P) = 112Hz<br>2J(P,P)=46Hz)<br>-3,7 (d.d.d,1J(Rh,P) = 113Hz<br>2J(PS,PR) = 46Hz<br>2J(PS,PR) = 31Hz) |
| 1H-NMR (200 MHz,$CDCl_3$): | 8,2-6,6(m,Ph), 3,7(m, = CH),<br>3,4(m,CH), 3,1(m,$CH_2$),<br>2,5-2,0(3xd,1J(P,H) = 14Hz,P-$CH_3$),<br>1,8-1,0(m,1J(H,H) = 7Hz<br>2J(Rh,H) = 140Hz,P-$CH_2$)<br>1,9-1,7(g,4J(P,H) = 10Hz,$PCH_3$) |
| IR (RbI-Pressing): | 3054 (m), 2987 (m), 2919 (m), 1433 (m) 1272 (s), 1221 (m), 1148 (s), 1097 (m) 1029 (s), 899 (s), 880 (s), 741 (m) 696 (m), 636 (m), 492 (m), 460 (m) |

c) α,α',α''-Tris(methylphenylphosphino)-neopentan-tris(acetonitril)-rhodium-(III)-tris-trifluormethansulfonat-Komplex

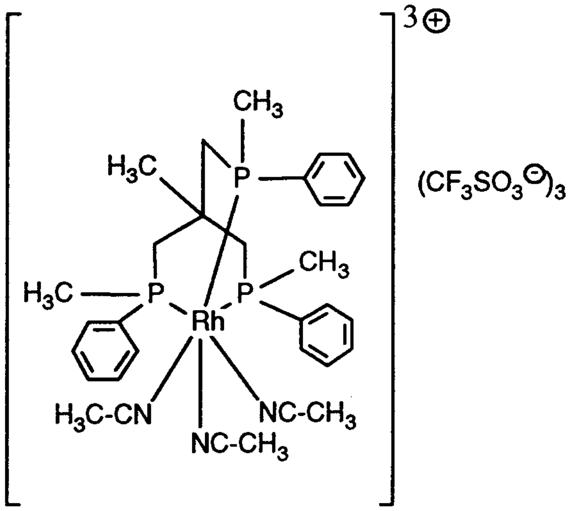

In einem 10 ml Kolben werden 100 mg des nach Beispiel H3c) erhaltenen Komplexes in 2 ml entgastem Acetonitril gelöst und für 10 Minuten mit Wasserstoffgas gesättigt. Mit einer Mikrospritze setzt man 126 µl Trifluormethansulfonsäure zu, worauf die orange Lösung gelb wird. Nach 15 Minuten Rühren wird der α,α',α''-Tris(methylphenylphosphino)-neopentan-tris(acetonitril)-rhodium-(III)-tris-trifluormethansulfonat-Komplex aus der Lösung mit entgastem Aether gefällt. Der weisse Niederschlag wird abzentrifugiert und mit Aether und Pentan gewaschen. Ausbeute: 132 mg analysenreiner Katalysator der Formel V

| Elementaranalyse berechnet für $C_{35}H_{42}N_3O_9F_9P_3S_3Rh$ (1111,73): | | | |
|---|---|---|---|
| | C: 37,81 | H: 3,81 | N: 3,78 |
| gefunden | C: 35,85 | H: 3,72 | N: 2,69 |

d) 26 g 2RS-2,3-Dihydroxy-1-(4-phenoxyphenoxy)-propan und 14,5 g Propionaldehyddiäthylacetal werden in 30 ml Toluol gelöst. Dieser Mischung fügt man 55 mg des nach Beispiel 3Hc) hergestellten Acetalisierungskatalysator zu und rührt das Gemisch für 50 Stunden bei Raumtemperatur. Man erhält so 29 g reines 2R,4S-2-Aethyl-4-[(4-phenoxyphenoxy)-methyl]-dioxolan mit einer optischen Reinheit von grösser als 90 % als farblose ölige Flüssigkeit mit einem Brechungsindex von $n_D^{20}$: 1,5459.

Beispiel H4: 2R-2,3-Dihydroxy-1-(4-phenoxyphenoxy)-propan

a) 4S-2,2-Dimethyl-4-[(4-phenoxyphenoxy)-methyl]-dioxolan

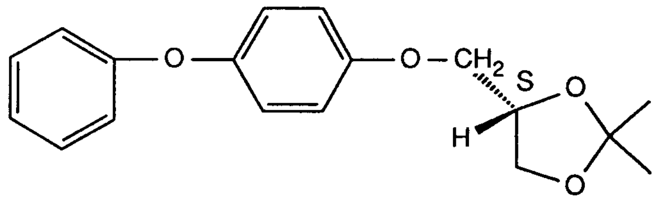

Die Lösung von 38,1 g 4-Phenoxyphenol in 350 ml wasserfreiem Dimethylsulfoxid wird unter Rühren und unter einer Stickstoffatmosphäre bei Raumtemperatur und leichter Aussenkühlung portionenweise mit insgesamt 25,3 g Kalium-tert.butylat versetzt. Zu der entstandenen grünen Lösung lässt man nun innerhalb von 1 Stunde bei Raumtemperatur die Lösung von 64,4 g D-α,β-Isopropylidenglycerin-γ-tosylat in 30 ml Dimethylsulfoxid zutropfen. Das Gemisch wird für 65 Stunden bei Raumtemperatur gerührt. Hierauf wird das Reaktionsgemisch auf 500 ml Eiswasser gegossen und wiederholt mit Diäthyläther extrahiert. Die vereinigten Aetherphasen werden wiederholt mit Wasser und gesättigter Natriumchloridlösung gewaschen, über Natriumsulfat getrockent und der Aether abdestilliert, wodurch das 4S-2,2-Dimethyl-4-[(4-phenoxyphenoxy)-methyl]-1,3-dioxolan als hellgelbes Oel erhalten wird, das nach einiger Zeit kristallin erstarrt, $[\alpha]_D^{20}$= +5,5° ($CHCl_3$)/Reinheit ee ~99 %, Smp. 36,5-37°C.

b) Zu einer Lösung von 63,9 g 4S-2,2-Dimethyl-4-[(4-phenoxyphenoxy)-methyl]-1,3-dioxolan in 500 ml reinem Methanol gibt man 60 g saures Ionenaustauschharz (DOWEX 50 W 8 $H^{\oplus}$-Form, 16-40 mesh) und rührt für 48 Stunden kräftig bei Raumtemperatur. Die Methanol-Lösung wird nun vom Ionenaustauscher abfiltriert, das Austauschharz wiederholt mit Methanol gewaschen und aus den vereinigten Methanollösungen das Lösungsmittel im Vakuum abdestilliert. Das Rohprodukt wird in 150 ml Essigsäureäthylester in der Wärme gelöst, heiss durch eine Glassfilternutsche filtriert, mit insgesamt 125 ml n-Hexan versetzt und durch Abkühlen zur Kristallisation gebracht. Die so erhaltenen farblosen Kristalle von 2R-2,3-Dihydroxy-1-(4-phenoxyphenoxy)-propan besitzen einen Smp. von 88,6-89,5°C, Reinheit: ee ~99 %, $[\alpha]_D^{20}$: -6,7±08° (Aethanol).

Formulierungsbeipsiele (% = Gewichtsprozent)

Beispiel F1: Spritzpulver

| | a) | b) | c) |
|---|---|---|---|
| Wirkstoff I | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Oktylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

Beispiel F2: Emulsions-Konzentrat

| | |
|---|---|
| Wirkstoff I | 10% |
| Oktylphenolpolyäthylenglykoläther (4-5 Mol AeO) | 3 % |
| Ca-Dodecylbenzolsulfonat | 3 % |
| Ricinusölpolyäthylenglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

Beispiel F3: Staubemittel

| | a) | b) |
|---|---|---|
| Wirkstoff I | 5 % | 8 % |
| Talkum | 95 % | - |
| Kaolin | - | 92 % |

Man erhält anwendungsfertige Staubemittel, indem der Wirkstoff mit dem Träger vermischt und auf einer geeigneten Mühle vermahlen wird.

Beispiel F4: Extruder-Granulat

| | |
|---|---|
| Wirkstoff I | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

Beispiel F5: Umhüllungs-Granulat

| | |
|---|---|
| Wirkstoff I | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

Beispiel F6: Suspensions-Konzentrat

| | |
|---|---|
| Wirkstoff I | 40 % |
| Äthylenglykol | 10 % |
| Nonylphenolpolyäthylenglykoläther (15 Mol AeO) | 6 % |
| Na-Ligninsulfonat | 10 % |
| Carboxymethylcellulose | 1 % |
| Silikonöl in Form einer 75 %igen wässrigen Emulsion | 1 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel B1: Wirkung gegen Nilaparvata lugens

Reispflanzen werden mit einer wässrigen Emulsions-Spritzbrühe, die 400 ppm des Wirkstoffs der Formel I enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Die Verbindung der Formel I zeigt eine gute Wirkung gegen Nilaparvata lugens in diesem Test.

Beispiel B2: Wirkung gegen Boophilus microplus

Vollgesogene adulte Zecken Weibchen werden auf eine PVC Platte aufgeklebt und mit einem Wattebausch überdeckt. Zur Behandlung werden die Testtiere mit 10 ml einer wässrigen Testlösung, die 125 ppm des Wirkstoffs der Formel I enthält, übergossen. Anschliessend wird der Wattebausch entfernt und die Zecken werden für 4 Wochen zur Eiablage inkubiert. Die Wirkung gegen Boophilus microplus zeigt sich entweder beim Weibchen als Mortalität oder Sterilität, oder bei den Eiern als ovizide Wirkung.

Die Verbindung der Formel I zeigt in diesem Test gute Wirkung gegen Boophilus microplus.

Beispiel B3: Ovizide Wirkung auf Adoxophyes reticulana

Auf Filterpapier abgelegte Eier von Adoxophyes reticulana werden für kurze Zeit in eine acetonisch-wässrige Testlösung, die 400 ppm des Wirkstoffs der Formel I enthält, eingetaucht. Nach dem Antrockenen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).

Die Verbindung der Formel I zeigt in diesem Test gute Wirkung gegen Adoxophyes reticulana.

Beispiel B4: Ovizide Wirkung auf Lobesia botrana

Auf Filterpapier abgelegte Eier von Lobesia botrana werden für kurze Zeit in eine acetonisch-wässrige Testlösung, die 400 ppm des Wirkstoffs der Formel I enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).

Die Verbindung der Formel I zeigt in diesem Test gute Wirkung gegen Lobesia botrana.

Beipsiel B5: Wirkung gegen Aonidiella aurantii

Kartoffelknollen werden mit Wanderlarven ("Crawlern") von Aonidiella aurantii (rote Citrus-Schildlaus) besiedelt. Nach etwa 2 Wochen werden die Kartoffeln in eine wässrige Emulsions- resp. Suspensions-Spritzbrühe getaucht, die den zu prüfenden Wirkstoff in einer Konzentration von 400 ppm enthält. Nach dem Abtrocknen der so behandelten Kartoffelknollen werden diese in einem Plastikbehälter inkubiert. Zur Auswertung wird 10-12 Wochen später die Überlebensrate der Wanderlarven der ersten Folgegeneration der behandelten Schildlaus-Population mit derjenigen der unbehandelten Kontrollansätze verglichen.

Die Verbindung der Formel I zeigte in diesem Test gute Wirkung gegen Aonidiella aurantii. Insbesondere wirkt die Verbindung der Formel I auch noch bei einer Konzentration von 0,1 ppm zu 100 %, während eine derartige vollständige Wirkung von vorbekanntem Enantiomerengemisch gleicher Struktur nur bei 0,75 ppm erreicht wird.

Beispiel B6: Ovizide Wirkung auf Heliothis virescens

Auf Filterpapier abgelegte Eier von Heliothis virescens werden für kurze Zeit in eine acetonisch-wässrige Testlösung, die 400 ppm des Wirkstoffs der Formel I enthält, eingetaucht. Nach dem Antrocknen der Testlösung werden die Eier in Petrischalen inkubiert. Nach 6 Tagen wird der prozuentuale Schlupf der Eier im Vergleich zu unbehandelten Kontrollansätzen ausgewertet (% Schlupfreduktion).

Die Verbindung der Formel I zeigt in diesem Test gute Wirkung gegen Heliothis virescens. Insbesondere zeigt diese Verbindung eine Wirkung 90 % auch noch bei 200 ppm, während das vorbekannte Enantiomerengemisch bei 400 ppm wirkungslos ist.

Beispiel B7: Phytotoxizitätstest

Baumwollpflanzen in 4-Blatt-Stadium werden mit wässrigen Suspensionen der zu prüfenden Verbindungen in Konzentrationen von 2000, 1000, 500 und 250 ppm besprüht. Nach Trocknen des Sprühbelags werden die behandelten Pflanzen im Gewächshaus kultiviert Nach 7 Tagen wird der Versuch durch Bewertung der Schäden in Prozent an den behandelten Pflanzen im Vergleich zu unbehandelten Kontrollpflanzen ausgewertet.

| Ergebnis: Testpflanze Baumwolle Auswertung 7 Tage nach Applikation | | |
|---|---|---|
| Konzentration ppm | Phytotoxizität in % | |
| | Wirkstoff Formel I | vorbekanntes Enantiomerengemisch gleicher Struktur |
| 2000 | 15 | 25 |
| 1000 | 5 | 15 |
| 500 | < 2,5 | 5 |
| 250 | < 2,5 | 0 |

In diesem Test zeigte die Verbindung der Formel I im Vergleich zum vorbekannten Enantiomerengemisch eine deutlich verringerte Schädigung der behandelten Kulturpflanzen. Gleiche Schadensbilder wurden vom vorbekannten Enantiomerengemisch jeweils schon bei der Hälfte der Wirkstoffkonzentration des erfindungsgemässen Einzelisomeren der Formel I erreicht.

**Patentansprüche**

**Patentansprüche für folgende Vertragsstaaten : AT, BE, CH, DE, FR, GB, GR, IT, LI, LU, NL, PT**

**1.** Die Verbindung 2R,4S-2-Aethyl-4-[(4-phenoxyphenoxy)-methyl]-dioxolan der Formel I

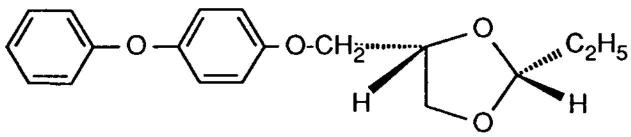

(I)

**2.** Verwendung der Verbindung der Formel I gemäss Anspruch 1 zur Bekämpfung von phytopathogenen Schädlingen.

**3.** Verfahren zur Bekämpfung von phytopathogenen Schädlingen, dadurch gekennzeichnet, dass man diese oder ihren Lebensraum mit einer wirksamen Menge der Verbindung der Formel I gemäss Anspruch 1 behandelt.

**4.** Schädlingsbekämpfungsmittel, welches als aktive Komponente die Verbindung der Formel I gemäss Anspruch 1 enthält.

**5.** Mittel gemäss Anspruch 4, dadurch gekennzeichnet, dass es neben dem Wirkstoff der Formel I noch mindestens einen Trägerstoff enthält.

**6.** Verfahren zur Bekämpfung von tier- und pflanzenschädigenden Insekten und Vertretern der Ordnung Akarina, dadurch gekennzeichnet, dass man die Schädlinge oder ihren Lebensraum mit einer wirksamen Menge der Verbindung der Formel I gemäss Anspruch 1 behandelt.

**7.** Verwendung der Verbindung der Formel I gemäss Anspruch 1 zur Bekämpfung von schädlichen Insekten und Vertretern der Ordnung Akarina.

**8.** Verwendung gemäss Anspruch 7, dadurch gekennzeichnet, dass es sich bei den Schädlingen um pflanzenschädigende Insekten handelt.

**9.** Verfahren zur Herstellung der Verbindung der Formel I, dadurch gekennzeichnet, dass man entweder

a) das Bromäthyldioxolan der Formel II

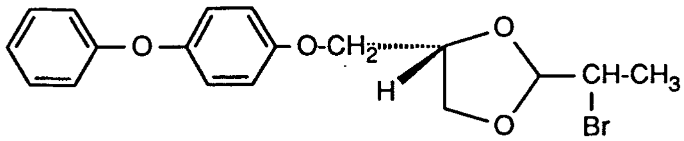

(II)

in Gegenwart von tert.Butanol und Kalium-tert.butylat dehydrobromiert, und das erhaltene Ketenacetal der Formel III

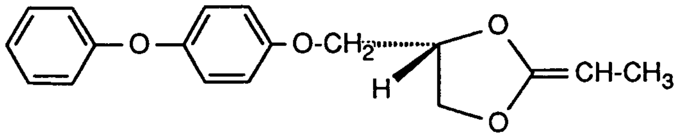

(III)

mit Wasserstoff in Gegenwart eines Palladium/Calciumcarbonat-Katalysators hydriert, oder

b) das Diol der Formel IV

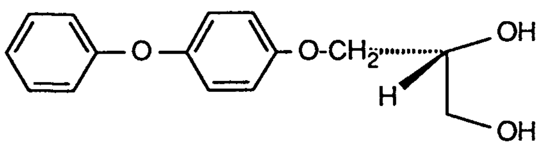

(IV)

in Gegenwart des stereoselektiven Katalysators der Formel V

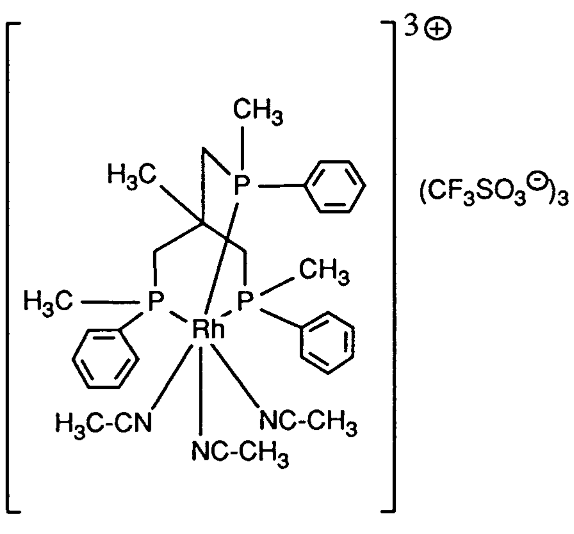

(V)

mit einem Propionaldehydacetal der Formel VI

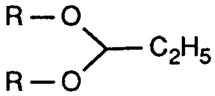

(VI)

worin R für $C_1$-$C_4$-Alkyl, vorzugsweise Methyl oder Aethyl steht, umsetzt.

**10.** Die Verbindung der Formel II

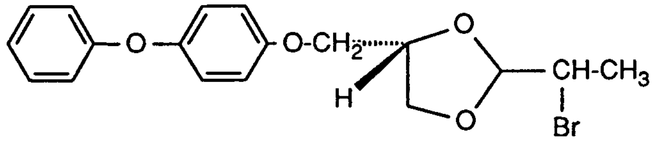

(II).

**11.** Die Verbindung der Formel III

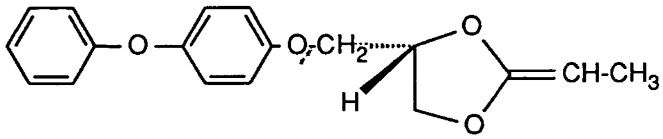

(III).

**12.** Die Verbindung der Formel IV

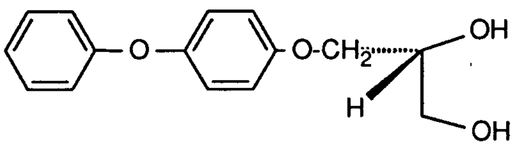

(V).

**13.** Die Verbindung der Formel X

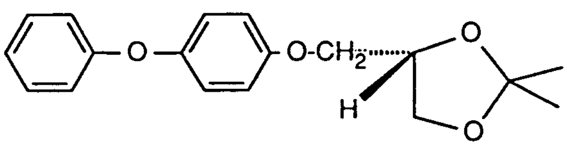

(X).

**14.** Die Verbindung der Formel XIV

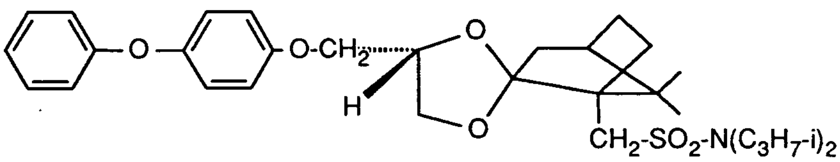

(XIV).

15. Die Verbindung der Formel V

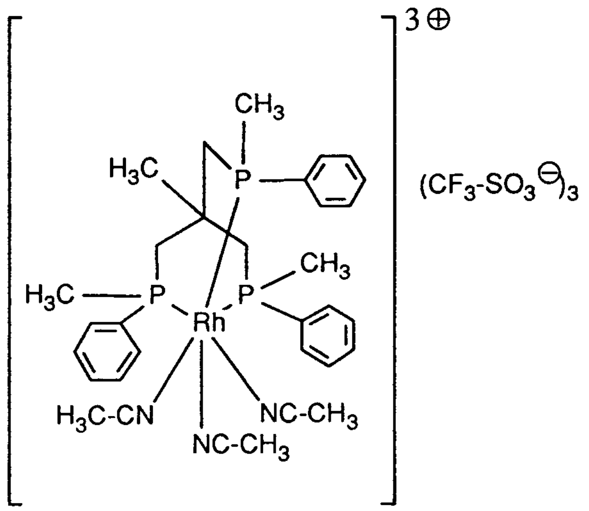

(V).

## Patentansprüche für folgenden Vertragsstaat : ES

1. Verwendung der Verbindung der Formel I

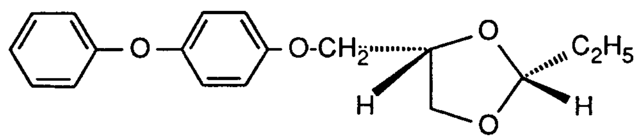

(I)

zur Bekämpfung von phytopathogenen Schädlingen.

2. Verfahren zur Bekämpfung von phytopathogenen Schädlingen, dadurch gekennzeichnet, dass man diese oder ihren Lebensraum mit einer wirksamen Menge der Verbindung der Formel I gemäss Anspruch 1 behandelt.

3. Schädlingsbekämpfungsmittel, welches als aktive Komponente die Verbindung der Formel I gemäss Anspruch 1 enthält.

4. Mittel gemäss Anspruch 3, dadurch gekennzeichnet, dass es neben dem Wirkstoff der Formel I noch mindestens einen Trägerstoff enthält.

5. Verfahren zur Bekämpfung von tier- und pflanzenschädigenden Insekten und Vertretern der Ordnung Akarina, dadurch gekennzeichnet, dass man die Schädlinge oder ihren Lebensraum mit einer wirksamen Menge der Verbindung der Formel I gemäss Anspruch 1 behandelt.

6. Verwendung der Verbindung der Formel I gemäss Anspruch 1 zur Bekämpfung von schädlichen Insekten und Vertretern der Ordnung Akarina.

7. Verwendung gemäss Anspruch 6, dadurch gekennzeichnet, dass es sich bei den Schädlingen um pflanzenschädigende Insekten handelt.

8. Verfahren zur Herstellung der Verbindung der Formel I, dadurch gekennzeichnet, dass man entweder

a) das Bromäthyldioxolan der Formel II

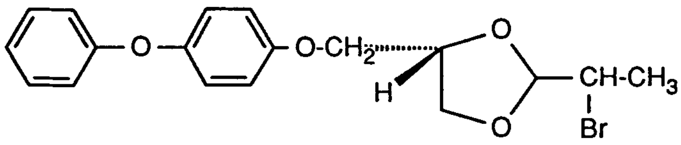

(II)

in Gegenwart von tert.Butanol und Kalium-tert.butylat dehydrobromiert, und das erhaltene Ketenacetal der Formel III

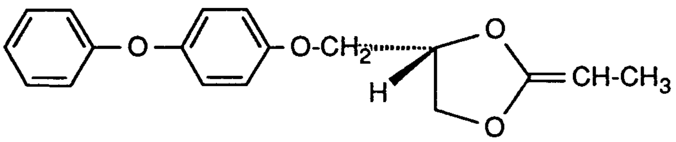

(III)

mit Wasserstoff in Gegenwart eines Palladium/Calciumcarbonat-Katalysators hydriert, oder

b) das Diol der Formel IV

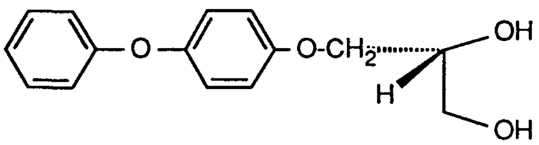

(IV)

in Gegenwart des stereoselektiven Katalysators der Formel V

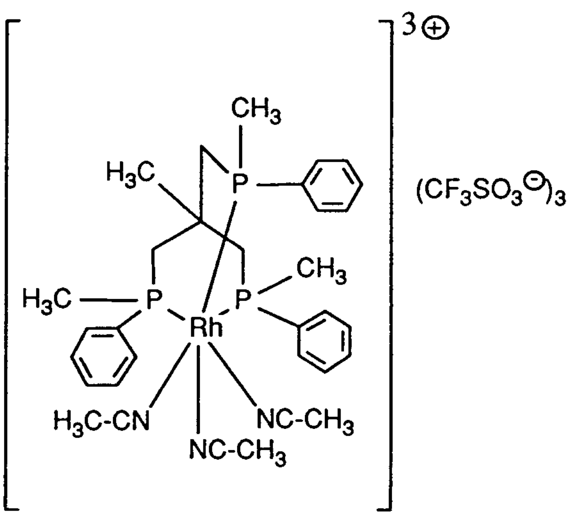

(V)

mit einem Propionaldehydacetal der Formel VI

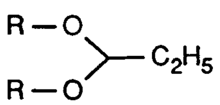

(VI)

worin R für $C_1$-$C_4$-Alkyl, vorzugsweise Methyl oder Aethyl steht, umsetzt.

## Claims

**Claims for the following Contracting States : AT, BE, CH, DE, FR, GB, GR, IT, LI, LU, NL, PT**

1. The compound 2R,4S-2-ethyl-4-[(4-phenoxyphenoxy)methyl]-dioxolan of formula I

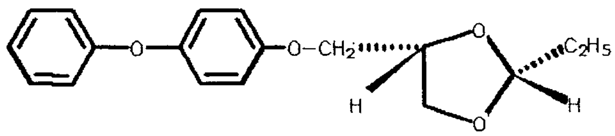

(I).

2. The use of the compound of formula I according to claim 1 for controlling phytopathogenic pests.

3. A method of controlling phytopathogenic pests, which comprises treating the pests or their locus with an effective amount of the compound of formula I according to claim 1.

4. A pesticidal composition comprising as active ingredient the compound of formula I according to claim 1.

5. A composition according to claim 4 comprising in addition to the compound of formula I at least one carrier.

6. A method of controlling insects and representatives of the order Acarina that are harmful to animals and plants, which comprises treating the pests or their locus with an effective amount of the compound of formula I according to claim 1.

7. The use of the compound of formula I according to claim 1 for controlling harmful insects and representatives of the order Acarina.

8. The use according to claim 7 wherein the pests are plant-destructive insects.

9. A process for the preparation of the compound of formula I, which comprises either

   a) dehydrobrominating the bromoethyldioxolan of formula II

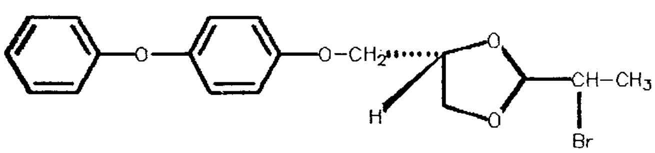

(II)

   in the presence of tert-butanol and potassium tert-butoxide, and hydrogenating the resulting ketene acetal of formula III

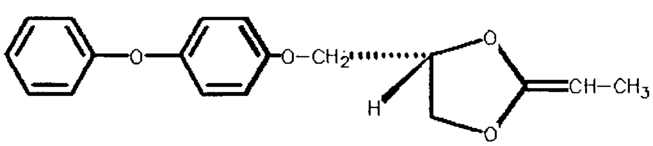

(III)

   with hydrogen in the presence of a palladium/calcium carbonate catalyst, or

b) reacting the diol of formula IV

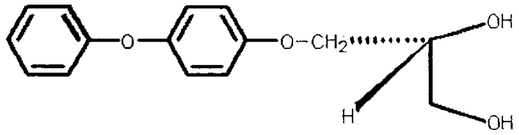

(IV)

in the presence of the stereoselective catalyst of formula V

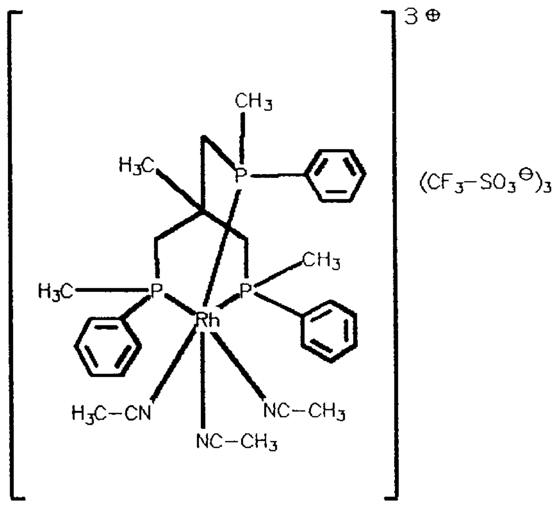

(V)

with a propionaldehyde acetal of formula VI

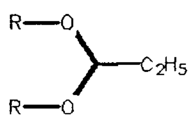

(VI)

wherein R is $C_1$-$C_4$alkyl, preferably methyl or ethyl.

**10.** The compound of formula II

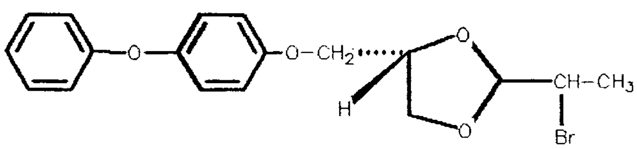

(II).

**11.** The compound of formula III

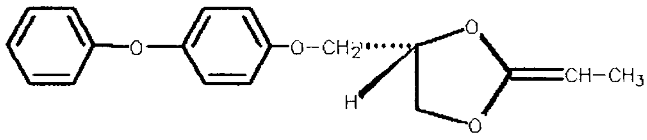

(III).

**12.** The compound of formula IV

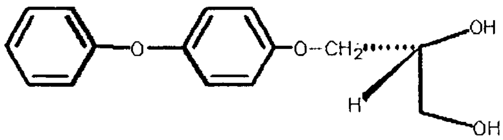

(V).

**13.** The compound of formula X

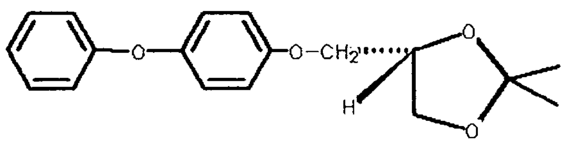

(X).

**14.** The compound of formula XIV

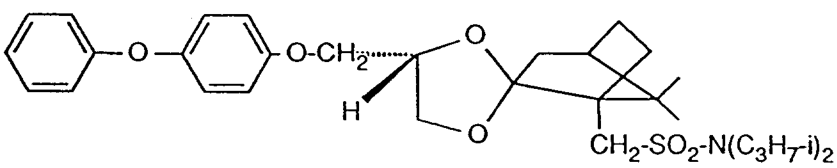

(XIV).

**15.** The compound of formula V

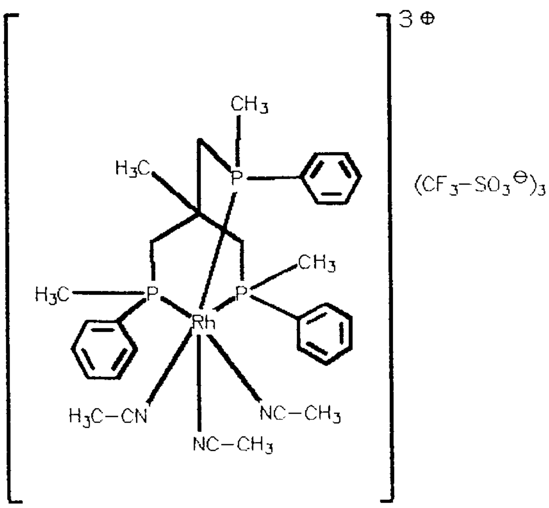

(V).

**Claims for the following Contracting State : ES**

**1.** The use of the compound of formula I

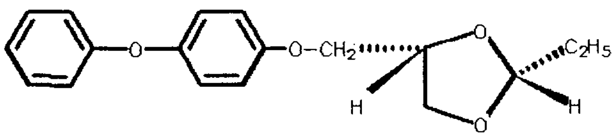

(I)

for controlling phytopathogenic pests.

**2.** A method of controlling phytopathogenic pests, which comprises treating the pests or their locus with an effective amount of the compound of formula I according to claim 1.

**3.** A pesticidal composition comprising as active ingredient the compound of formula I according to claim 1.

**4.** A composition according to claim 3 comprising in addition to the compound of formula I at least one carrier.

**5.** A method of controlling insects and representatives of the order Acarina that are harmful to animals and plants, which comprises treating the pests or their locus with an effective amount of the compound of formula I according to claim 1.

**6.** The use of the compound of formula I according to claim 1 for controlling harmful insects and representatives of the order Acarina.

**7.** The use according to claim 6 wherein the pests are plant-destructive insects.

**8.** A process for the preparation of the compound of formula I, which comprises either

a) dehydrobrominating the bromoethyldioxolan of formula II

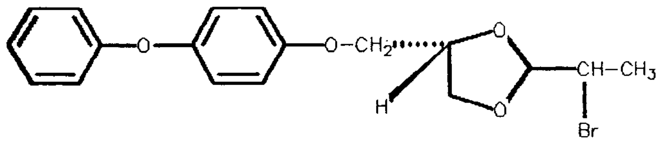

(II)

in the presence of tert-butanol and potassium tert-butoxide, and hydrogenating the resulting ketene acetal of formula III

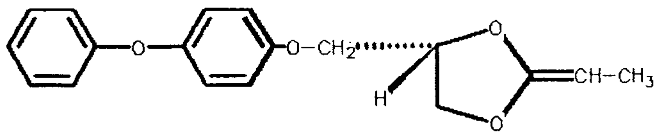

(III)

with hydrogen in the presence of a palladium/calcium carbonate catalyst, or

b) reacting the diol of formula IV

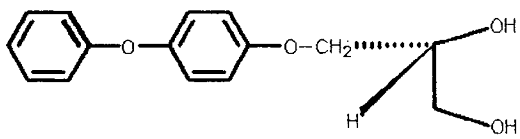

(IV)

in the presence of the stereoselective catalyst of formula V

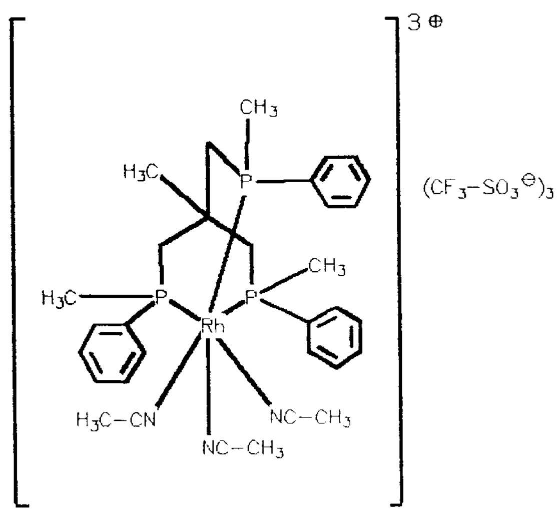

(V)

with a propionaldehyde acetal of formula VI

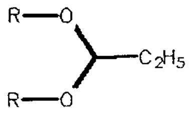

(VI)

wherein R is $C_1$-$C_4$alkyl, preferably methyl or ethyl.

## Revendications

**Revendications pour les Etats contractants suivants : AT, BE, CH, DE, FR, GB, GR, IT, LI, LU, NL, PT**

1. Le composé 2R,4S-2-éthyl-4-[(4-phénoxyphénoxy)|méthyl]dioxolanne de formule I

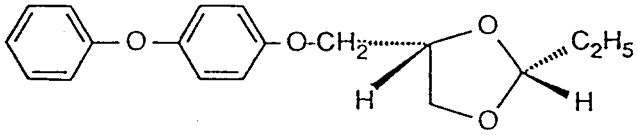

(I)

2. Utilisation du composé de formule I de la revendication 1 pour la lutte contre les parasites phytopathogènes.

3. Procédé pour combattre les parasites phytopathogènes, caractérisé en ce que l'on traite ces parasites ou leur habitat par une quantité efficace du composé de formule I de la revendication 1.

4. Produit parasiticide contenant en tant que composant actif le composé de formule I de la revendication 1.

5. Produit, selon revendication 4, caractérisé en ce que, avec la substance active de formule I, il contient encore au moins un véhicule.

6. Procédé pour combattre les insectes et représentants de l'ordre des acariens qui sont nuisibles pour les animaux et les végétaux, caractérisé en ce que l'on traite les parasites ou leur habitat par une quantité efficace du composé de formule I de la revendication 1.

7. Utilisation du composé de formule I de la revendication 1 pour la lutte contre les insectes et représentants de l'ordre des acariens qui sont nuisibles.

8. Utilisation selon revendication 7, caractérisée en ce que les parasites sont des insectes nuisibles pour les végétaux.

9. Procédé de préparation du composé de formule I, caractérisé en ce que

   a) on soumet le brométhyldioxolanne de formule II

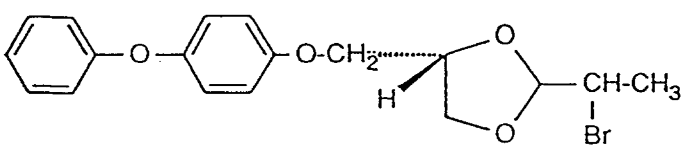

(II)

à déshydrobromuration en présence de tert-butanol et de tert-butylate de potassium, ce qui donne le cétène-acétal de formule III

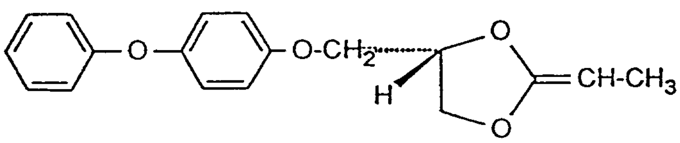

(III)

qu'on hydrogène en présence d'un catalyseur au palladium sur carbonate de calcium, ou bien

b) on fait réagir le diol de formule IV

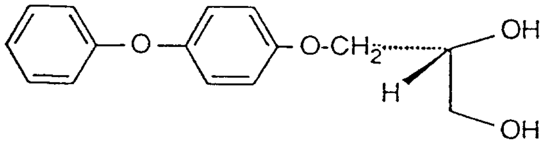

(IV)

en présence du catalyseur stéréosélectif de formule V

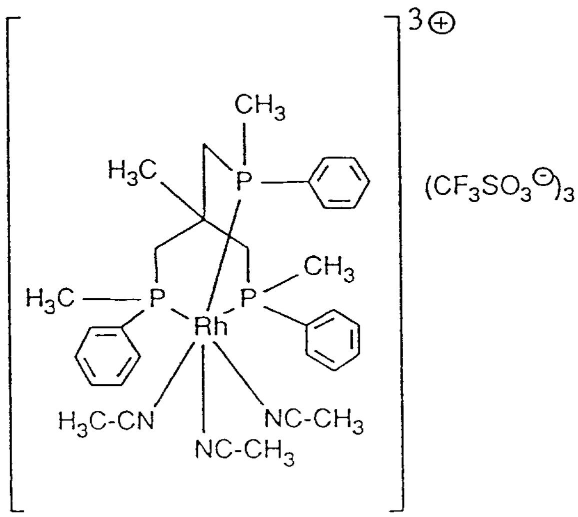

(V)

avec un acétal du propionaldéhyde de formule VI

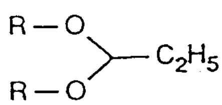

(VI)

dans laquelle R représente un groupe alkyle en C1-C4, de préférence méthyle ou éthyle.

**10.** Le composé de formule II

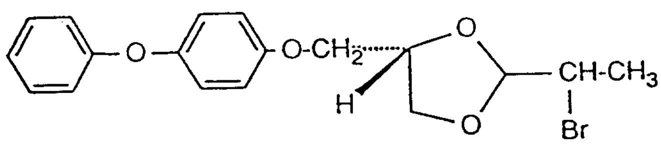

(II).

**11.** Le composé de formule III

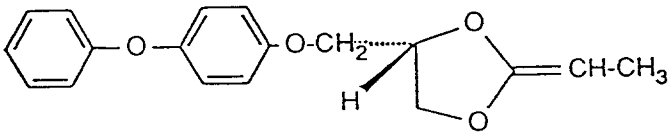

(III).

**12.** Le composé de formule IV

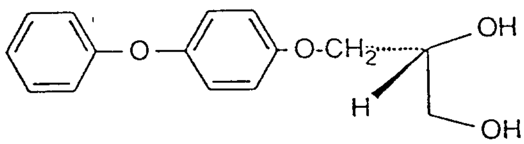

(IV).

**13.** Le composé de formule X

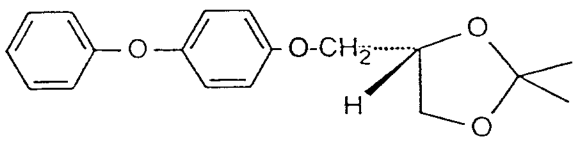

(X).

**14.** Le composé de formule XIV

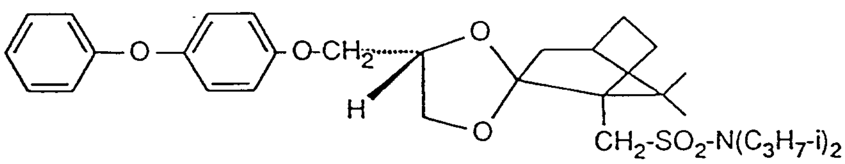

(XIV).

15. Le composé de formule V

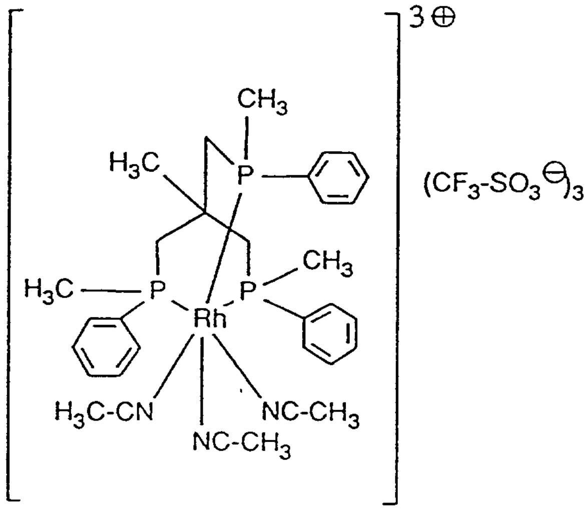

(V).

**Revendications pour l'Etat contractant suivant : ES**

1. Utilisation du composé de formule I

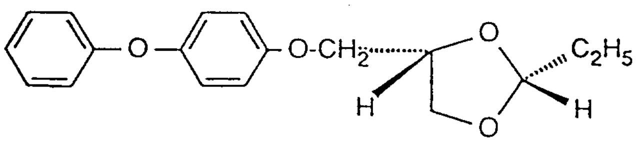

(I)

pour la lutte contre les parasites phytopathogogènes.

2. Procédé pour combattre les parasites phytopathogogènes, caractérisé en ce que l'on traite ces parasites ou leur habitat par une quantité efficace du composé de formule I de la revendication 1.

3. Produit parasiticide contenant en tant que composant actif, le composé de formule I de la revendication 1.

4. Produit selon revendication 3, caractérisé en ce que, avec la substance active de formule I, il contient encore au moins un véhicule.

5. Procédé pour combattre les insectes et représentants de l'ordre des acariens nuisibles pour les animaux et les végétaux, caractérisé en ce que l'on traite les parasites ou leur habitat par une quantité efficace du composé de formule I de la revendication 1.

6. Utilisation du composé de formule I de la revendication 1 pour la lutte contre les insectes et représentants de l'ordre des acariens qui sont nuisibles.

7. Utilisation selon revendication 6, caractérisée en ce que les parasites en question sont des insectes nuisibles pour les végétaux.

8. Procédé de préparation du composé de formule I, caractérisé en ce que

a) on soumet le brométhyldioxolanne de formule II

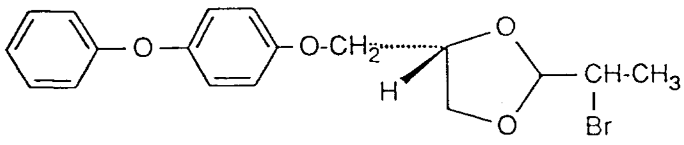

(II)

à déshydrobromuration en présence de tert-butanol et de tert-butylate de potassium, ce qui donne le cétène-acétal de formule III

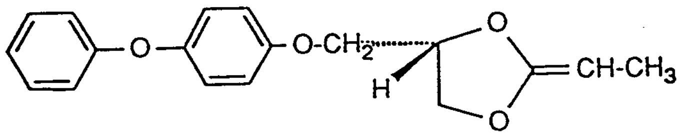

(III)

qu'on hydrogène en présence d'un catalyseur au palladium sur carbonate de calcium,
ou bien
b) on fait réagir le diol de formule IV

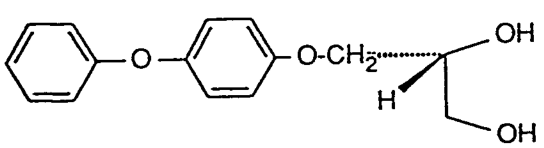

(IV)

en présence du catalyseur stéréosélectif de formule V

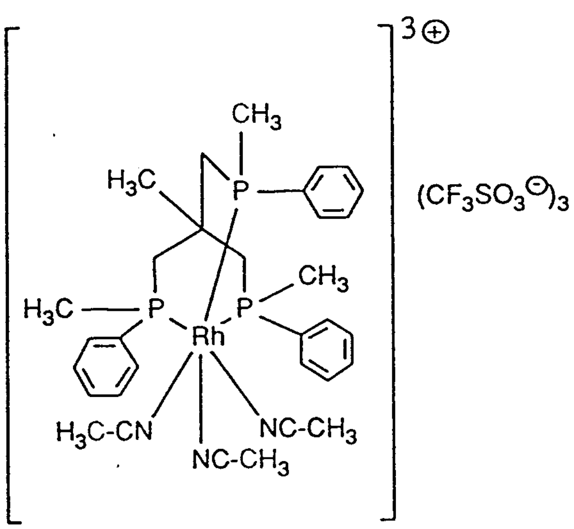

(V)

avec un acétal du propionaldéhyde de formule VI

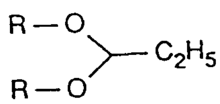

(VI)

dans laquelle R représente un groupe alkyle en C1-C4, de préférence méthyle ou éthyle.